# EUROPEAN PATENT APPLICATION

(11) **EP 1 795 190 A1**
(43) Date of publication of application: **13.06.2007**
(21) Application number: 06256209.5
(22) Date of filing: 06.12.2006
(51) Int. Cl.: A61K 31/122, A61P 3/00

(54) **Agent for suppressing body fat accumulation**

(30) Priority: 07.12.2005 JP 2005354170
(71) Applicant: YAMAHA HATSUDOKI KABUSHIKI KAISHA, Iwata-shi, Shizuoka 438-8501 (JP)
(72) Inventor: Okada, Yumika, Iwata-shi Shizuoka 438-8501 (JP); Iio, Kumiko, Iwata-shi Shizuoka 438-8501 (JP)
(74) Representative: Schlich, George William

(57) **Abstract**

Novel and highly effective agents for suppressing fat accumulation and for suppressing body fat gain that contain astaxanthin and/or an ester thereof as an active component are provided. The agent for suppressing fat accumulation according to the present invention effectively suppresses accumulation of fat both in subcutaneous fat and in visceral fat. Even when high-fat diet intake is continued, body weight gain is suppressed by using the agent for suppressing body weight gain according to the present invention. Therefore, life-style related diseases, which are very likely to be caused by obesity, can be prevented, and the agents can be also used as an agent for preventing or alleviating a disease or a symptom having a relation to fat accumulation.

## Description

### BACKGROUND OF THE INVENTION

### Field of the Invention

The present invention relates to an agent for suppressing body fat accumulation. More specifically, the present invention relates to an agent for suppressing body fat accumulation and an agent for suppressing body weight gain, the agents containing astaxanthin and/or an ester thereof as an active component.

### Description of the Related Art

Obesity refers to not only the extent of body weight, but also to a state in which the ratio of fat in the body (body fat) is high, that is, a state in which the number of hypertrophic adipocytes is increased or a state in which adipocytes themselves are enlarged. Among adipocytes, there are white adipocytes that store excess calories from overeating as fat and brown adipocytes that store fat also, but then act to release energy. Of these, it is the white adipocytes that relate to obesity. Preadipocytes, which are precursors of white adipocytes, proliferate and differentiate into white adipocytes which store fat droplets within the cells.

Obesity is caused when caloric expenditure is lower than caloric intake and the energy source that thus has not been expended accumulates as body fat. The causes of body fat accumulation due to excess energy include lack of exercise, improper eating habit, stress, lipid metabolism abnormality (disorder), excessive secretion of insulin, enlargement of adipocytes, and lack of brown adipocytes.

Obesity due to such causes is a risk factor for various life-style related diseases such as diabetes, hypertension, and hyperlipemia, and it is important to first prevent obesity in order to prevent such life-style related diseases. As obesity types, there are subcutaneous fat obesity and visceral fat obesity. Subcutaneous fat is stored just under the skin, and the number of subcutaneous adipocytes increases easily. On the other hand, visceral fat is stored in the mesentery located within the peritoneal cavity, and visceral adipocytes tend to store fat within the individual cells. For subcutaneous fat obesity, the incidence of life-style related diseases is not high, whereas for visceral fat obesity, the risk of developing life-style related diseases is very high.

As methods for reducing obesity, exercise therapy, dietary therapy, and drug therapy can be used. Moreover, various health food products having an inhibitory action on the digestion and absorption also are commercially available. However, each of these methods is difficult to follow on a continuous basis, has side effects, and is associated with other problems.

Carotenoids are naturally-occurring substances having an antioxidative effect, and their various bioactivities have attracted interest. However, few studies have been conducted to investigate the action of carotenoids on obesity and adipocytes. It has been reported only that a carotenoid derived from a vegetable or a fruit suppresses the differentiation induced by insulin of preadipocytes into adipocytes (Japanese Laid-Open Patent Publication No. 2003-95930). However, obesity, as discussed above, is caused not only by an increase in the number of adipocytes but also, especially in visceral fat obesity, by accumulation of fat within adipocytes. Moreover, it has been reported also that differentiation of preadipocytes into adipocytes is accompanied by induction of expression of adiponectin, but when differentiation is impaired and thus fat atrophies, adiponectin becomes deficient, causing a metabolic disorder, which leads to obesity (Takashi Kadowaki et al., "The Role of Adiponectin in Molecular Mechanisms of Diabetes and Cardiovascular Diseases", proceedings of The 128th Japanese Association of Medical Sciences Symposium on "Diabetes Mellitus and Atherosclerosis", December 2, 2004, pp. 34-45). Therefore, since carotenoids merely suppress differentiation into adipocytes, it is doubtful whether carotenoids have a sure anti-obesity action.

### SUMMARY OF THE INVENTION

It is an object of the present invention to provide safe and highly effective agents for suppressing body fat accumulation and for suppressing body weight gain.

The present invention provides an agent for suppressing body fat accumulation, the agent comprising astaxanthin and/or an ester thereof as an active component.

In one embodiment, the fat is subcutaneous fat and/or visceral fat.

The present invention also provides an agent for suppressing body weight gain, the agent comprising astaxanthin and/or an ester thereof as an active component.

In an embodiment, the astaxanthin and/or the ester thereof is derived from a microalga belonging to the genus *Haematococcus.*

The present invention further provides an agent for preventing or alleviating a disease or a symptom having a relation to fat accumulation, the agent comprising astaxanthin and/or an ester thereof as an active component.

In addition, the present invention provides a use of astaxanthin and/or an ester thereof in the manufacture of an agent for suppressing body fat accumulation.

In one embodiment, the fat is subcutaneous fat and/or visceral fat.

The present invention further provides a use of astaxanthin and/or an ester thereof in the manufacture of an agent for suppressing body weight gain.

The present invention also provides a use of astaxanthin and/or an ester thereof in the manufacture of an agent for preventing or alleviating a disease or a symptom having a relation to fat accumulation.

In addition to the above, the present invention also provides pharmaceutical compositions and health food supplements comprising astaxanthin and/or an ester thereof as an active component.

The invention also provides a method of manufacturing an agent as defined herein, the method comprising (a) growing in culture a microalga belonging to the genus *Haematococcus*; and (b) extracting and purifying astaxanthin and/or an ester thereof from the culture.

According to the present invention, novel and highly effective agents for suppressing fat accumulation and for suppressing body weight gain are provided. The agent for suppressing fat accumulation according to the present invention effectively suppresses accumulation of fat both in subcutaneous fat obesity and in visceral fat obesity. Even when a high-fat diet intake is continued, body weight gain is suppressed by using the agent for suppressing body weight gain according to the present invention. Therefore, life-style related diseases, which are very likely to be caused by obesity, can be prevented, and a disease or a symptom having a relation to fat accumulation can be prevented or alleviated. Furthermore, the agent for suppressing fat accumulation according to the present invention has very low toxicity and thus offers a high degree of safety.

### BRIEF DESCRIPTION OF THE DRAWINGS

Fig. 1 is a graph showing the change over time in body weight of mice during a period of 16 weeks from the start of a test.
Fig. 2 is a graph showing the weights of subcutaneous fat and visceral fat of the mice in each group at the end of the test.
Fig. 3 is a graph showing the proportions of the weights of various adipose tissues and the liver to body weight of the mice in each group at the end of the test.

### DESCRIPTION OF THE PREFERRED EMBODIMENTS

Astaxanthin or an ester thereof used in the present invention is a carotenoid represented by the following formula: 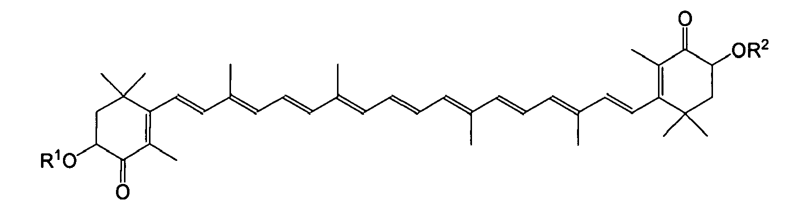 wherein R¹ and R² are both hydrogen in the case of astaxanthin, and R¹ and R² are each independently a hydrogen atom or a fatty acid residue provided that at least one of R¹ and R² is a fatty acid residue in the case of an ester of astaxanthin. Examples of the fatty acid residue in the ester of astaxanthin include, but are not limited to, saturated fatty acids such as palmitic acid and stearic acid or unsaturated fatty acids such as oleic acid, linoleic acid, α-linolenic acid, γ-linolenic acid, bishomo-γ-hnolenic acid, arachidonic acid, eicosapentaenoic acid, and docosahexaenoic acid, or any combination thereof. The astaxanthin ester of the present invention can be any mono- or diester, homogeneous or non-homogeneous. Astaxanthin has a structure in which an additional oxo group and an additional hydroxy group are present at each end of a β-carotene molecule, so that unlike for β-carotene, the stability of the molecule is low. On the other hand, an ester form (e.g., as obtained in an extract from krill) in which the hydroxy groups at both ends are esterified with an unsaturated fatty acid is more stable.

Astaxanthin or an ester thereof used in the present invention may be chemically synthesized or derived from a naturally-occurring product. Examples of the naturally-occurring products in the latter case include red yeast; the shell of crustaceans such as Tigriopus (red water flea) and krills; and microalgae such as green algae, which contain astaxanthin and/or an ester thereof. In the present invention, any extract containing astaxanthin and/or esters thereof produced by any method can be used. Generally, extracts from those naturally-occurring products can be used, and the extracts may be crude or purified if necessary. In the present invention, a crude extract or a crushed powder of naturally-occurring products, or a purified product or a chemically synthesized product, if necessary, that contains such astaxanthin and/or esters thereof can be used either alone or in combination. In view of the chemical stability, an ester form of astaxanthin is preferably used.

Since the agent for suppressing body fat accumulation according to the present invention also suppresses body weight gain by suppressing accumulation of fat in adipocytes, it can be used also as an agent for suppressing body weight gain. Furthermore, it can be used also as an agent for preventing or alleviating a disease or a symptom having a relation to fat accumulation. Examples of the disease or the symptom having a relation to fat accumulation include various life-style related diseases such as hyperlipemia, arteriosclerosis, hypertension, myocardial infarction, cerebrovascular disorders, cerebral infarction, angina pectoris, pancreatitis, diabetes, fatty liver, and metabolic disorders.

The agent for suppressing body fat accumulation, the agent for suppressing body weight gain, or the agent for preventing or alleviating a disease or a symptom having a relation to fat accumulation according to the present invention may be administered to a mammal subject in need of such suppression of body fat accumulation, in need of such suppression of body weight gain, or in need of prevention or alleviation of such disease or symptom. The mammal subject includes a human subject and a pet subject such as a dog, a cat, a rabbit, a hamster.

The route of administration of the agent for suppressing body fat accumulation, the agent for suppressing body weight gain, or the agent for preventing or alleviating a disease or a symptom having a relation to fat accumulation according to the present invention may be either oral or parenteral. The dosage form is selected appropriately according to the route of administration. Examples thereof include parenteral solutions, infusion solutions, powders, granules, tablets, capsules, pills, enteric-coated preparations, troches, liquids for internal use, suspensions, emulsions, syrups, liquids for external use, poultices, nose drops, ear drops, eye drops, inhalants, ointments, lotions, suppositories, and enteral nutrients. These can be used either alone or in combination depending on the condition of a disease. To prepare these dosage forms, auxiliary substances commonly used in the field of pharmaceutical manufacturing technology, such as excipients, binders, antiseptics, antioxidants, disintegrators, lubricants, and flavoring agents, can be used as necessary.

The dose of the agent for suppressing body fat accumulation, the agent for suppressing body weight gain, or the agent for preventing or alleviating a disease or a symptom having a relation to fat accumulation according to the present invention varies depending on the purpose of administration, the individual to be administrated (sex, age, body weight, etc.), and the severity and nature of the disease, and can be determined by a person skilled in the art. Usually, the dose for an adult in terms of free or unesterified form of astaxanthin may be 0.1 mg to 2 g, preferably 1 mg to 1 g, more preferably 4 mg to 500 mg per day in the case of oral administration, while it may be 0.01 mg to 1 g, preferably 0.015 mg to 750 mg, more preferably 0.1 mg to 500 mg per day in the case of parenteral administration.

The agent for suppressing body fat accumulation, the agent for suppressing body weight gain, or the agent for preventing or alleviating a disease or a symptom having a relation to fat accumulation according to the present invention can be used not only as pharmaceuticals as described above, but also as the category of products regulated as "quasi-drugs", cosmetics, food products, nutritional supplements, foods and drinks, and other similar products. When used as quasi-drugs or cosmetics, the agent may be used in conjunction with various auxiliary substances commonly used in the field of quasi-drugs or cosmetics, or other technologies, if necessary. Alternatively, when used as food products, nutritional supplements, or foods and drinks, the agent may be used in conjunction with additives commonly used for food products, for example, sweeteners, spices, seasonings, antiseptics, preservatives, germicides, and antioxidants, if necessary. The agent may be used in a desired form such as solution, suspension, syrup, granule, cream, paste, or jelly, or may be shaped, if necessary. The ratio of the agent contained in these products is not particularly limited, and can be selected appropriately according to the intended purpose, the mode of usage, and the amount of usage.

### Examples

### Preparation Example 1: Preparation of Astaxanthin Monoester

An astaxanthin monoester was prepared in the following manner. *Haematococcus pluvialis* K0084 strain was cultivated at 25°C under irradiation with light while bubbling a gas containing 3% CO₂ into the medium and under nutrient stress condition (i.e. nitrogen source deprivation), and then was encysted. The encysted cells were disrupted by a bead beater, and a lipophilic fraction was extracted with ethanol. The extract contained lipids such as triglyceride in addition to astaxanthins. The extract was subjected to column chromatography using a synthetic resin adsorbent to give a purified product containing astaxanthin monoesters. This purified product was analyzed by HPLC, and it was confirmed that this purified product contained an astaxanthin monoester having a molecular weight of 858 as the main component, did not contain the free form of astaxanthin, the diester form of astaxanthin, and triglyceride, and that it contained a small amount of diglyceride.

### Example 1: Effect of astaxanthin monoester on obese model mice fed with a high-fat diet

Astaxanthin was administered to obese model mice fed with a high-fat diet, and the change in body weight, the amount of subcutaneous fat (in the inguinal region and the back), the amount of visceral fat (around the reproductive organs and around the kidney), and the liver weight were examined in the following manner.

Four week old male C57BL/6J strain mice (SPF) purchased from CHARLES RIVER LABORATORIES JAPAN, INC. were used. The mice were preliminarily fed for 8 days and used for the test after they reached the age of 5 weeks. The mice were divided into three groups of 8 each, that is, a normal diet group, a high-fat diet group, and a high-fat diet + astaxanthin (AX) group, so that the average body weight was equal among the groups.

During the preliminary feeding period, the mice were given an ordinary powder diet (MF, Oriental Yeast Co., Ltd.), and during the test period of 16 weeks, the mice were given the ordinary powder diet or a high-fat diet having the composition shown in Table 1 below. As to drinking water, the mice were allowed to drink freely sterile distilled water from a water supply bottle.

**Table 1**

| Component | Composition of high-fat diet (part by weight) |
|---|---|
| Beef tallow | 400 |
| Corn starch | 100 |
| Glucose | 90 |
| AIN- 76TM mineral mix | 40 |
| AIN-76TM vitamin mix | 10 |
| Casein | 360 |

| | |
|---|---|
| AIN-76 compositions from Oriental Yeast Co., Ltd. | |

The astaxanthin monoester prepared in Preparation Example 1 was dissolved in an olive oil (Wako Pure Chemical Industries, Ltd.) to prepare a solution containing astaxanthin monoester at a concentration of 60 mg/mL. This solution was administered to the high-fat diet + AX group and the olive oil to the other two groups in a volume of 0.05 mL/10 g body weight every day for 16 weeks from the start of the test (at the age of 5 weeks) to the age of 21 weeks, using a probe for oral administration in the mice.

During the test period, the body weight was measured once a week using a scale. At the end of the test, the body weight was measured, and thereafter, the mice were fasted overnight and sacrificed by collecting blood from the heart. Then, the liver, the adipose tissue in the inguinal region, the adipose tissue around the reproductive organs, the adipose tissue around the kidney, and the adipose tissue in the back were collected and the weights thereof were measured.

The different types of data obtained were expressed as average values ± standard errors for each group. In order to test for statistically significant differences between the high-fat diet group and the high-fat diet + AX group or the normal diet group, a multiple comparison test (ANOVA) was performed using an analysis software (Stat View, Abacus Inc., USA), and a comparison between the groups was performed using Fisher's PLSD multiple comparison test. Differences were considered statistically significant when p < 0.05.

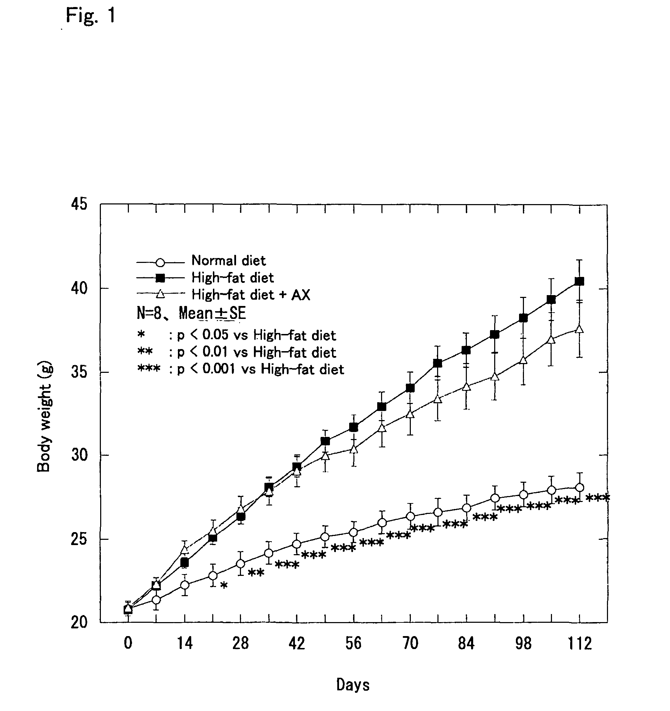

Fig. 1 shows the change over time in mean body weight of the mice during the period of 16 weeks from the start of the test. Body weight in the high-fat diet group significantly increased when compared to the normal diet group. Body weight in the high-fat diet + AX group increased more than the normal diet group, but the increase in body weight tended to be distinctly suppressed more than in the high-fat diet group.

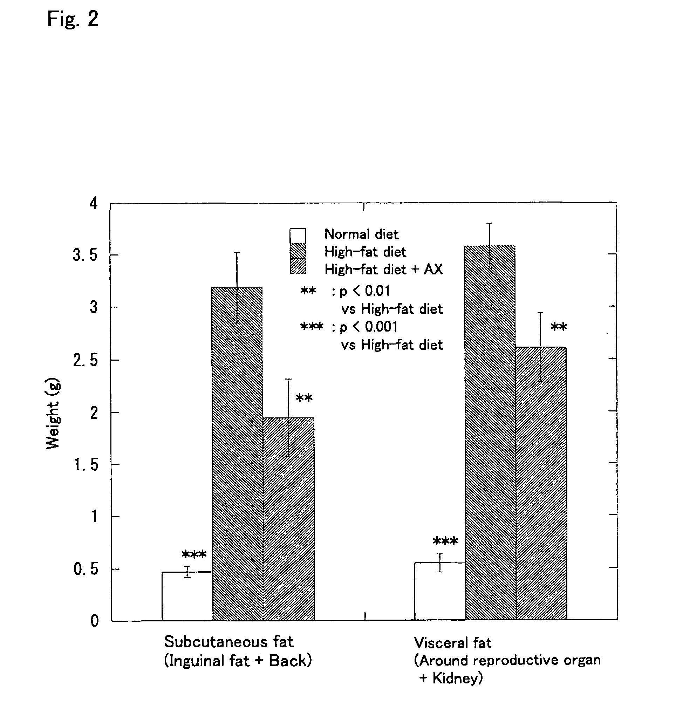

Fig. 2 shows the mean weights of subcutaneous fat and visceral fat of the mice in each group. It can be seen from Fig. 2 that although the amount of fat accumulation both in subcutaneous fat and in visceral fat was considerably increased by intake of the high-fat diet, the accumulation of fat was significantly suppressed when taking astaxanthin together with the high-fat diet.

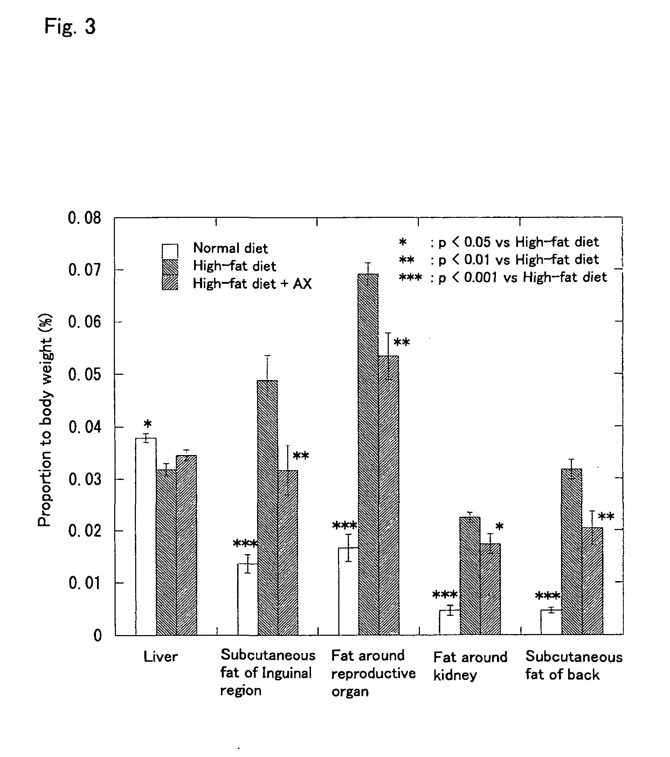

Fig. 3 shows the proportions of the mean weights of the adipose tissues and the liver to mean body weight. The proportions of all of the adipose tissues to body weight were dramatically increased by intake of the high-fat diet, but the increase was significantly suppressed when taking astaxanthin together with the high-fat diet. Moreover, it was found that although the proportion of the liver weight to body weight was reduced by intake of the high-fat diet, the proportion approached that in the case of the normal diet when taking astaxanthin together with the high-fat diet.

### Reference Example 1: Measurement of 50% Lethal Concentration for HUVEC

Human umbilical vein endothelial cells (HUVECs) (ATCC CRL-1730) were obtained from American Type Culture Collection and precultivated in an Endothelial Cell Growth Medium (CELL APPLICATIONS, USA) containing 10% bovine fetal serum supplemented with 1% Antibiotic-Antimycotic (GIBCO BRL, USA) under a 5% CO₂ atmosphere at 37°C.

A Matrigel matrix (BD Biosciences, USA) was melted and kept at 4°C on ice, and then, 50 µL of the matrix were transferred to each well of a 96-well tissue culture plate. The plate was incubated at 37°C for at least one hour to solidify the matrix solution.

On the other hand, the astaxanthin monoester obtained in Preparation Example 1 was dissolved in dimethylsulfoxide (DMSO), and then diluted with distilled water to prepare stock test solutions in which the astaxanthin monoester was contained in 40 (v/v)% DMSO at 25000, 2500, 250, 25, and 2.5 µM, respectively.

Next, 100 µL of a HUVEC suspension (about 2.5 × 10³ cells/well) were poured into the 96-well Matrigel plate under a 5% CO₂ atmosphere at 37°C. After 24 hours, 100 µL of a growth medium and 2 µL of each of the stock test solutions or the vehicle (40 (v/v)% DMSO) were added to two wells each, and incubated for an additional 72 hours. The final concentrations of the astaxanthin monoester were 250, 25, 2.5, 0.25, and 0.025 µM.

After the incubation, 20 µL of a 90% alamarBlue reagent were added to individual wells, and incubated for an additional 6 hours. Then, the fluorescence intensity of each well was measured at an excitation wavelength of 530 nm and an emission wavelength of 590 nm using a Spectrafluor Plus plate reader to count the number of living cells. This measurement is based on the ability of a living cell to change alamarBlue from the non-fluorescent, oxidized form (blue) to the fluorescent, reduced form (red). The 50% lethal concentration was calculated as the concentration at which the number of living cells was 50% of the number of cells at the start of the experiment.

The result indicates that the 50% lethal concentration (LC₅₀) of the astaxanthin monoester for the HUVECs was 250 µM (maximum concentration of the astaxanthin monoester dissolved in DMSO) or more, and thus it was found that the toxicity of the astaxanthin monoester is low.

According to the present invention, novel agents for suppressing body fat accumulation and for suppressing body weight gain are provided. The agent for suppressing body fat accumulation and the agent for suppressing body weight gain can be useful not only for preventing obesity but also for preventing or alleviating various life-style related diseases having an apparent relation to fat accumulation, such as hyperlipemia, arteriosclerosis, hypertension, myocardial infarction, cerebrovascular disorders, cerebral infarction, angina pectoris, pancreatitis, diabetes, fatty liver, and metabolic disorders. Moreover, even when high-fat diet intake is continued, body weight gain is suppressed by using the agent for suppressing body weight gain according to the present invention. Therefore, even in dietary therapy for obesity, severe restrictions are not necessary so that it is easy to continue with the therapy.

Astaxanthin and/or an ester thereof, which is an active component in the agent for suppressing body fat accumulation, the agent for suppressing body weight gain, or the agent for preventing or alleviating a disease or a symptom having a relation to fat accumulation according to the present invention, has been consumed in food for a long time and has low toxicity. Therefore, astaxanthin and/or an ester thereof has a very high degree of safety. Accordingly, these agents are not only used as pharmaceuticals, but can be used also prophylactically on a daily basis as health food products.

The invention may be embodied in other forms without departing from the spirit or essential characteristics thereof. The embodiments disclosed in this application are to be considered in all respects as illustrative and not limiting. The scope of the invention is indicated by the appended claims rather than by the foregoing description, and all changes which come within the meaning and range of equivalency of the claims are intended to be embraced therein.

## Claims

1. An agent for suppressing body fat accumulation, e.g. in a human, comprising astaxanthin and/or an ester thereof as an active component.

2. The agent for suppressing body fat accumulation of claim 1, wherein the fat is subcutaneous fat and/or visceral fat.

3. The agent for suppressing body fat accumulation of claim 1 or 2, wherein the astaxanthin and/or the ester thereof is derived from a microalga belonging to the genus *Haematococcus.*

4. An agent for suppressing body weight gain, e.g. in a human, comprising astaxanthin and/or an ester thereof as an active component.

5. The agent for suppressing body weight gain of claim 4, wherein the astaxanthin and/or the ester thereof is derived from a microalga belonging to the genus *Haematococcus.*

6. An agent for preventing or alleviating a disease or a symptom having a relation to fat accumulation, e.g. in a human, comprising astaxanthin and/or an ester thereof as an active component.

7. Use of astaxanthin and/or an ester thereof in the manufacture of an agent for suppressing body fat accumulation, e.g. in a human.

8. The use according to claim 7, wherein the fat is subcutaneous fat and/or visceral fat.

9. Use of astaxanthin and/or an ester thereof in the manufacture of an agent for suppressing body weight gain, e.g. in a human.

10. Use of astaxanthin and/or an ester thereof in the manufacture of an agent for preventing or alleviating a disease or a symptom having a relation to fat accumulation, e.g. in a human..

11. The agent of claim 5 or the use according to claim 10, wherein the disease or symptom is selected from the group consisting of hyperlipemia, arteriosclerosis, hypertension, myocardial infarction, cerebrovascular disorders, cerebral infarction, angina pectoris, diabetes, fatty liver, and metabolic disorders.

12. The agent of any one of claims 1 to 6, or the use according to any one of claims 7 to 10, wherein the agent comprises an ester of astaxanthin, e.g. a monoester.

13. A pharmaceutical composition comprising astaxanthin and/or an ester thereof as an active component.

14. A health food supplement comprising astaxanthin and/or an ester thereof as an active agent.

15. A method of manufacturing an agent of any one of claims 1 to 6, comprising:
(a) growing in culture a microalga belonging to the genus *Haematococcus*; and
(b) extracting and purifying astaxanthin and/or an ester thereof from the culture.
